# NEUE EUROPÄISCHE PATENTSCHRIFT

(11) **EP 0 699 055 B2**
(45) Veröffentlichungstag und Bekanntmachung des Hinweises auf die Entscheidung über den Einspruch: **19.09.2001**
(45) Hinweis auf die Patenterteilung: 01.07.1998
(21) Anmeldenummer: 94913558.6
(22) Anmeldetag: 08.04.1994
(51) Int. Cl.: A61B 17/70, A61B 17/80

(54) **OSTEOSYNTHESEVORRICHTUNG**
OSTEOSYNTHESIS DEVICE
DISPOSITIF POUR OSTEOSYNTHESE

(30) Priorität: 18.05.1993 DE 4316542
(43) Veröffentlichungstag der Anmeldung: 06.03.1996
(60) Teilanmeldung: 97110013.6
(73) Patentinhaber: SCHÄFER micomed GmbH, 73035 Göppingen (DE)
(72) Erfinder: HALM, Henry, D-49143 Bissendorf-Wissingen (DE); REHDER, Günther, D-73650 Winterbach (DE); SCHÄFER, Bernd, D-73614 Schorndorf (DE)
(74) Vertreter: Dreiss, Fuhlendorf, Steimle & Becker
(86) Internationale Anmeldenummer: EP9401095
(87) Internationale Veröffentlichungsnummer: WO9426191

(56) Entgegenhaltungen:
- EP-A- 0 384 001
- EP-A- 0 528 706
- DE-A- 3 916 198
- DE-A- 4 107 480
- DE-C- 4 110 002
- FR-A- 2 624 720
- GB-A- 2 178 323
- US-A- 4 653 481

## Beschreibung

Die Erfindung betrifft eine Osteosynthesevorrichtung mit einer Knochenschraube oder einer Pedikelschraube gemäß dem Oberbegriff des Anspruchs 1.

Derartige Osteosynthesevorrichtungen sind in einer Vielzahl bekannt (DE-Gbm 90 04 240, DE-Gbm 91 04 027, EP-A-346 521, DE-A 39 42 429, EP-A-443 894, EP-A-348 272, EP-A-465 158, EP-A-528 706, EP-A-443 892, DE-A 39 16 198, DE-C 41 10 002). Aus diesen Druckschriften sind unterschiedliche Knochenschrauben mit Gabelköpfen bekannt, in die ein Korrekturstab eingelegt und fixiert werden kann. Dabei kommt es hauptsächlich auf eine rutschfreie Verankerung des Korrekturstabes an der Knochenschraube an. Nur eine rutsch- und verdrehsichere Fixierung des Korrekturstabes an der Knochenschraube gewährleistet eine optimale Übertragung von Zug- und Druckkräften auf die einzelnen zu korrigierenden und fixierenden Knochen sowie die Übertragung von Dreh- und Biegemomenten.

In der Regel wird eine gute Fixierung dadurch erzielt, daß in den Gabelkopf eine Fixierschraube derart eingeschraubt wird, daS diese auf den eingelegten Korrekturstab drückt. Es hat sich jedoch gezeigt, daß, aufgrund der auftretenden hohen Kräfte und Momente, ein Aufbiegen des Gabelkopfes nicht auszuschließen ist, so daß sich die Fixierung des Korrekturstabes lösen kann. Außerdem bietet eine derartige Klemmfixierung keine ausreichende Sicherheit gegen ein Verdrehen des Korrekturstabes um die eigene Achse.

Bei einem klemmenden Osteosyntheseimplantat (EP-A-528 706) wird auf den in den Gabelkopf der Knochenschraube eingelegten Korrekturstab eine Fixierplatte aufgelegt, der einerseits die Aufgabe hat, daß die beiden Schenkel des Gabelkopfes nicht auseinandergepreßt werden, andererseits an seinem auf dem Korrekturstab aufliegenden Abschnitt Längsnuten aufweist, die in Längsnuten des Korrekturstabes eingreifen. Hierdurch wird zwar eine erhöhte Sicherheit gegen ein Verdrehen des Korrekturstabes im Gabelkopf erzielt, jedoch muß das gesamte am Korrekturstab wirkende Drehmoment über die Andruckplatte, eine Fixierschraube und die beiden Schenkel des Gabelkopfes auf die Knochenschraube übertragen werden. Bei hohen Drehmomenten besteht immer noch die Gefahr, daß der Gabelkopf verformt und dadurch die Fixierung des Korrekturstabes gelockert wird.

Aus der GB 2 178 323 A ist eine Haltevorrichtung bekannt, mit der der Korrekturstab zwischen zwei Haltebacken gespannt wird. Die Haltebacken weisen Längskerben auf, in die der längsgekerbte Stab eingelegt wird. Aus der FR 2 624 720 A1 ist eine Knochenschraube mit einer Hutmutter bekannt geworden, bei der die Hutmutter zur Anlage an einen geriffelten Stab gebracht wird.

Ausgehend von der FR- A- 2 624 720 liegt der Erfindung die Aufgabe zugrunde, eine Osteosynthesevorrichtung der eingangs genannten Art derart weiterzubilden, daß der Korrekturstab sicher gegen ein Verdrehen an der Knochenschraube festlegbar ist.

Diese Aufgabe wird erfindungsgemäß durch die Merkmale des kennzeichnenden Teils des Anspruchs 1 gelöst.

Die am Nutgrund des Gabelkopfes, d.h. am Grund der Nut, in die der Korrekturstab eingelegt wird, vorgesehene Kerben, nachfolgend Längskerben genannt, erlauben einerseits eine verdrehsichere Befestigung des Korrekturstabes an der Knochenschraube, andererseits eine direkte Kraftübertragung vom Korrekturstab auf den Schaft der Knochenschraube. Wird ein Korrekturstab mit einer glatten Oberfläche in den Gabelkopf eingelegt, dann graben sich die Längskerben in die Oberfläche des Korrekturstabes ein und fixieren diesen auf diese Weise. Ist der Korrekturstab als Gewindestange ausgebildet, dann graben sich die Längskerben in das Gewinde der Gewindestange ein. Die Längskerben verlaufen in Richtung der Achse des Korrekturstabes.

Die erfindungsgemäße Ausgestaltung der Knochenschraube hat den Vorteil, daß die Kraftübertragung vom Korrekturstab auf den Schaft der Knochenschraube nicht über Halteelemente, Schrauben oder die beiden Schenkel des Gabelkopfes erfolgt, sondern direkt vom Stab auf den Schaft. Auf diese Weise können wesentlich höhere Kräfte und Momente übertragen werden bzw. wird die Kraft bzw. das Moment mit höherer Sicherheit gegen Verrutschen bzw. Durchrutschen übertragen.

Vorteilhaft ist der Korrekturstab mit Längsnuten versehen, wobei die Längsnuten zu den Längskerben korrespondieren und insbesondere in Achsrichtung verlaufen. Bei dieser Ausführungsform greifen die Längsnuten beim Einlegen des Korrekturstabes in die Nut des Gabelkopfes in die Längskerben und stellen eine formschlüssige Verbindung zwischen dem Korrekturstab und der Knochenschraube her. Dabei können sowohl der Korrekturstab als auch der Gabelkopf aus einem harten formbeständigen Material bestehen. Hartes bzw. zähes Material ist insbesondere bei der Übertragung hoher Kräfte und Momente empfehlenswert.

Ein problemloses Einlegen und anschließendes Entfernen des Korrekturstabes in die Nut des Gabelkopfes wird dadurch erzielt, daß die Längskerben in Richtung der Achse der Knochenschraube hinterschnittsfrei ausgebildet sind. Da die Längskerben keine Hinterschnitte aufweisen, kann der Korrekturstab auch nach einer Verformung beim Einpressen in die Längskerben problemlos wieder aus der Nut des Gabelkopfes entfernt werden, ohne daß er sich in den Längskerben festkrallt.

Bevorzugt sind die Längskerben am Rand des Gabelkopfes vorgesehen. Dies hat den Vorteil, daß aufgrund der kürzeren Längskerben wesentlich höhere Druckkräfte beim Anpressen des Korrekturstabes erzielt werden können, so daß sich die Längskerben bei einem glatten Korrekturstab wesentlich besser in dessen Oberfläche eingraben können. Außerdem wird durch den höheren Anpreßdruck die Haltekraft sowohl bei glatten als auch bei genuteten Korrekturstäben erhöht.

Bevorzugt ist der Gabelkopf der Knochenschraube mit einem Außengewinde, insbesondere für eine Kopfmutter, versehen. Durch diese Kopfmutter, die nach dem Einlegen des Korrekturstabes in den Gabelkopf auf diesen aufgeschraubt wird, wird verhindert, daß sich die beiden Schenkel des Gabelkopfes bei hoher Krafteinwirkung auseinanderbiegen. Ferner kann durch die Kopfmutter der Korrekturstab in den Grund des Gabelkopfes gedrückt werden.

Bei einer Weiterbildung ist vorgesehen, daß die Kopfmutter ein Innengewinde mit einem Zentrierbund aufweist. Über diesen Zentrierbund wird das Aufsetzen der Kopfmutter auf die Gabelmutter wesentlich erleichtert, so daß das Aufschrauben der Kopfmutter auch in schwer zugänglichen Lagen, wie sie bei operativen Eingriffen stets vorkommen, dennoch, z.B. bei ventraler Lager der Knochenschraube, problemlos und schnell möglich ist.

Bei einem Ausführungsbeispiel ist die Kopfmutter als Hutmutter ausgebildet und weist ein koaxiales Innengewinde für eine Fixierschraube auf. Bei diesem Ausführungsbeispiel wird der Korrekturstab über die Fixierschraube in den Grund des Gabelkopfes gedrückt.

Bei einer Weiterbildung ist vorgesehen, daß die Fixierschraube am eingeschraubten Ende eine Ringschneide aufweist und insbesondere als Innensechskantschraube ausgebildet ist. Über die Ringschneide gräbt sich beim Festziehen der Fixierschraube das eingeschraubte Ende in den im Gabelkopf sich befindenden Bereich des Korrekturstabes und hält diesen zusätzlich gegen Verrutschen oder Verdrehen fest.

Vorteilhaft ist die axiale Stirnfläche der Kopfmutter, d.h. die Oberseite der Kopfmutter, ballig, insbesondere kugelig, ausgebildet und insbesondere von einem Sechskant umgeben. Über den Sechskant kann die Kopfmutter auf einfache Weise auf den Gabelkopf der Knochenschraube aufgeschraubt und festgezogen werden. Die ballige Form hat den Vorteil, daß es zu geringeren Irritationen des benachbarten Gewebes kommt.

Bei einem bevorzugten Ausführungsbeispiel weist die Knochenschraube einen sich von der Schraubenspitze in Richtung des Gabelkopfes konisch erweiternden Schraubenkern auf. Die konische Form des Schraubenkerns hat den Vorteil, daß die Knochenschraube besser im Knochen fixierbar ist, insbesondere beim Einschrauben in den Knochen stramm sitzt. Die Knochenschraube wird nicht nur über die Gewindegänge gehalten, sondern auch durch die Klemmwirkung des sich konisch erweiternden Schraubenkerns.

Vorzugsweise ist der Außen- bzw. Nenndurchmesser der Knochenschraube, d.h. der Gewindedurchmesser, konstant. Dabei nimmt vorteilhaft die Breite der Gewindeschneide der Knochenschraube von der Schraubenspitze ausgehend zu. Auch hierdurch wird eine Klemmwirkung erzielt, durch die Knochenschraube zusätzlich am Knochen festgehalten wird. Dabei ist bevorzugt die Gewindesteigung konstant.

Mit Vorzug ist das an den Schraubenkern sich anschließende Ende des Gabelkopfes ballig, insbesondere kugelig ausgebildet. Durch die ballige bzw. kugelige Form wird der Vorteil erzielt, daß die Knochenschraube auch bei schräger bzw. geneigter Anordnung auf einer Knochenplatte, insbesondere im distalen Bereich dennoch satt in der Aufnahmekalotte der Knochenplatte anliegt.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im einzelnen dargestellt ist. Dabei können die in der Zeichnung dargestellten und in der Beschreibung erwähnten Merkmale jeweils einzeln für sich oder in beliebiger Kombination bei der Erfindung verwirklicht sein. In der Zeichnung zeigen:
- Figur 1: eine Seitenansicht der erfindungsgemäßen Knochenschraube;
- Figur 2: eine Seitenansicht in Richtung des Pfeils II gemäß Figur 1, teilweise aufgeschnitten;
- Figur 3: eine Draufsicht auf die Knochenschraube;
- Figur 4: einen Längsschnitt durch eine Kopfmutter;
- Figur 5: eine Draufsicht auf die Kopfmutter;
- Figur 6: eine Explosionsdarstellung des oberen Teils der Knochenschraube mit einzulegendem Korrekturstab, Kopfmutter und Fixierschraube; und
- Figur 7: eine Zusammenbaudarstellung der Elemente gemäß Figur 6.

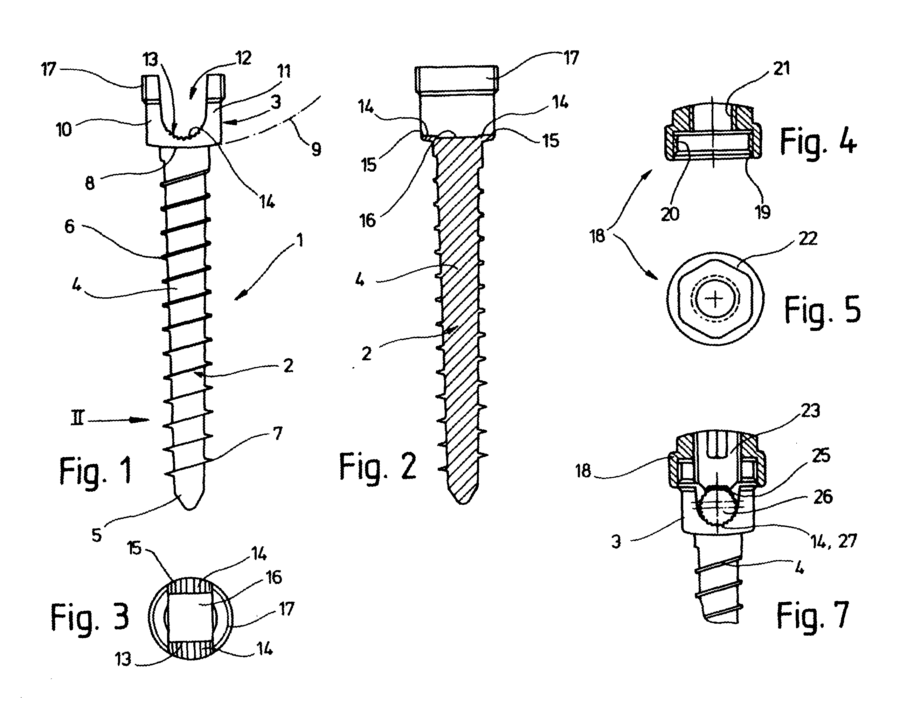

In der Figur 1 ist insgesamt mit 1 eine Knochenschraube, insbesondere eine Pedikelschraube dargestellt, die einen Gewindeschaft 2 und einen Gabelkopf 3 aufweist. Der Gewindeschaft 2 besteht aus einem Schraubenkern 4 mit Schraubenspitze 5 und einem den Schraubenkern 4 umgebenden Gewinde 6. Dabei ist erkennbar, daß der Schraubenkern 4 sich von der Schraubenspitze 5 in Richtung des Gabelkopfes 3 konisch erweitert. Der Durchmesser des Gewindes 6 bleibt jedoch konstant, wohingegen die Breite der Gewindeschneide 7 ausgehend von der Schraubenspitze 5 in Richtung des Gabelkopfes 3 zunimmt. Die Steigung des Gewindes 6 bleibt jedoch über die ganze Länge des Gewindeschaftes 2 konstant. Ferner ist in Figur 1 erkennbar, daß die Unterseite 8 des Gabelkopfes 3 ballig, insbesondere kugelförmig ausgebildet ist, was auch durch die Linie 9 andeutungsweise dargestellt sein soll.

Der Gabelkopf 3 weist zwei Schenkel 10 und 11 auf, zwischen denen eine Nut 12 eingearbeitet ist. Die Nut 12 besitzt sich geringfügig konisch öffnende Nutwände. Der Grund 13 der Nut 12 ist mit Längskerben 14 versehen, wie auch aus Figur 3 ersichtlich ist. Ferner ist erkennbar, daß die Längskerben 14 hinterschneidungsfrei in den Nutgrund 13 eingearbeitet sind. Wie aus den Figuren 1 bis 3 ersichtlich, befinden sich die Längskerben 14 am Nutrand 15, wohingegen der zentrale Bereich des Nutbundes frei von Längskerben ist. Die Längskerben 14 werden entweder durch Einfräsen oder durch Einschlagen gebildet.

Das obere Ende des Gabelkopfes 3 ist mit einem Außengewinde 17 versehen, welches sich über etwa ein Drittel der Höhe des Gabelkopfes 3 erstreckt. Auf dieses Außengewinde 17 ist eine Kopfmutter 18 (Figur 4) aufschraubbar, die als Hutmutter ausgebildet ist. Die Kopfmutter 18 weist an ihrem unteren Ende einen Zentrierbund 19 auf, an den sich ein Innengewinde 20 anschließt. Koaxial zum Innengewinde 20 ist die Kopfmutter 18 mit einem weiteren Innengewinde 21 versehen, welches einen kleineren Nenndurchmesser aufweist. Die Oberseite der Kopfmutter 18 ist ebenfalls ballig bzw. kugelig ausgebildet. Wie sich aus Figur 5 ergibt, weist die Oberseite der Kopfmutter 18 einen Außensechskant 22 auf, über den die Kopfmutter 18 auf die Knochenschraube 1 aufgedreht und festgezogen werden kann.

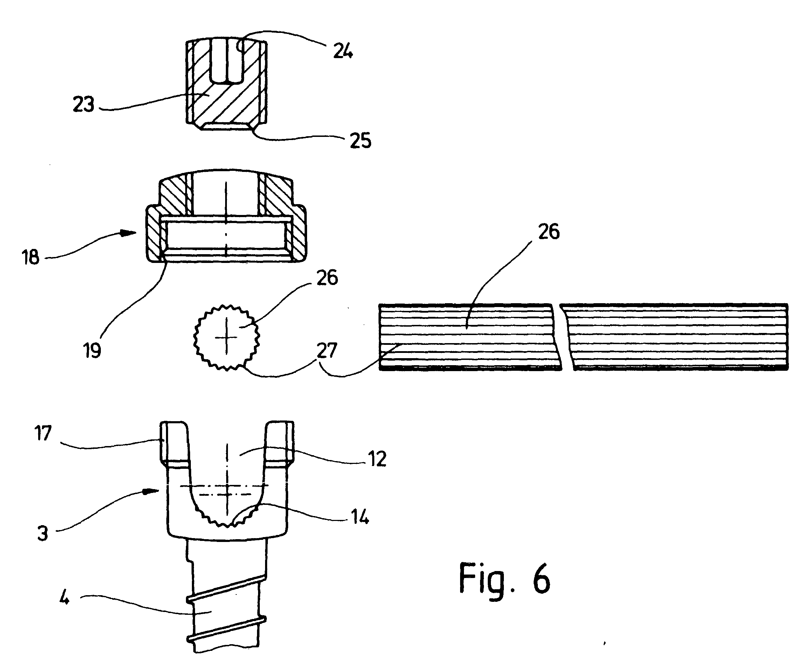

In das Innengewinde 21 ist eine Fixierschraube 23 (Figur 6) einschraubbar. Die Fixierschraube 23 ist an ihrer Oberseite ebenfalls ballig ausgeführt und weist einen Innensechskant 24 auf. An der Unterseite ist die Fixierschraube 23 mit einer Ringschneide 25 versehen, die bei angezogener Fixierschraube 23 auf einen Korrekturstab 26 drückt. Dieser Korrekturstab 26 (Figur 6) ist mit Längsnuten 27 versehen, die zu den Längskerben 14 des Nutgrundes 13 korrespondieren.

Wird der Korrekturstab 26, wie in Figur 6 dargestellt, in die Nut 12 des Gabelkopfes 3 eingelegt, dann greifen die Längsnuten 27 in die Längskerben 14 formschlüssig ein. Anschließend wird die Kopfmutter 18 aufgeschraubt, wobei der Zentrierbund 19 ein Ansetzen der Kopfmutter 18 am Außengewinde 17 wesentlich erleichtert. Schließlich wird die Fixierschraube 23 eingedreht und festgezogen, wobei sich die Ringschneide 25 in die benachbarten Längsnuten 27 eingräbt. Der Korrekturstab 26 ist auf diese Weise über die in die Längskerben 14 eingreifen Längsnuten 27 gegen Verdrehung und über die in die Längsnuten 27 eingegrabene Ringschneide 25 gegen Verschiebung gesichert. Außerdem werden Drehmomente vom Korrekturstab 26 direkt über die formschlüssige Verbindung mit den Längskerben 14 in den Schraubenkern 4 eingeleitet (Figur 7).

## Patentansprüche

1. Osteosynthesevorrichtung mit einer Knochenschraube (1) oder einer Pedikelschraube, mit einem eine Nut (12) aufweisenden Gabelkopf (3), einem in der Nut (12) des Gabelkopfes (3) gelagerten Korrekturstab (26) und einem Außengewinde (17) für eine Kopfmutter (18) am Gabelkopf/ (3), **dadurch gekennzeichnet, daß** der Grund (13) der Nut (12) des Gabelkopfes (3) mit mehreren parallel zueinander liegenden, in Längsrichtung der Nut (12) sich erstreckenden Kerben (14) versehen ist, wobei die Kerben (14) in Richtung der Achse der Knochenschraube (1) hinterschneidungsfrei ausgebildet sind.

2. Osteosynthesevorrichtung nach Anspruch 1, **dadurch gekennzeichnet, daß** die Kerben (14) am Rand (15) des Nutgrundes des Gabelkopfes (3) vorgesehen sind.

3. Osteosynthesevorrichtung nach einem der vorhergehenden Ansprüche, **dadurch gekennzeichnet, daß** der Korrekturstab (26) mit Längsnuten (27) versehen ist, wobei die Längsnuten (27) zu den Längskerben (14) korrespondieren und in Achsrichtung verlaufen.

4. Osteosynthesevorrichtung nach einem der vorhergehenden Ansprüche, **dadurch gekennzeichnet, daß** die Kopfmutter (18) ein Innengewinde (20) mit einem Zentrierbund (19) aufweist.

5. Osteosynthesevorrichtung nach einem der vorhergehenden Ansprüche, **dadurch gekennzeichnet, daß** die Kopfmutter (18) als Hutmutter ausgebildet ist und ein koaxiales Innengewinde (21) für eine Fixierschraube (23) aufweist.

6. Osteosynthesevorrichtung nach Anspruch 5, **dadurch gekennzeichnet, daß** die Fixierschraube (23) am eingeschraubten Ende eine Ringschneide (25) aufweist und z.B. als Innensechskantschraube ausgebildet ist.

7. Osteosynthesevorrichtung nach einem der vorhergehenden Ansprüche, **dadurch gekennzeichnet, daß** die axiale Stirnfläche der Kopfmutter (18) ballig oder kugelig ausgebildet und z.B. von einem Sechskant (22) umgeben ist.

8. Osteosynthesevorrichtung nach einem der vorhergehenden Ansprüche, **dadurch gekennzeichnet, daß** die Knochenschraube (1) einen sich von der Schraubenspitze (S) in Richtung des Gabelkopfes (3) konisch erweiternden Schraubenkern (4) aufweist.

9. Osteosynthesevorrichtung nach einem der vorhergehenden Ansprüche, **dadurch gekennzeichnet, daß** der Außen- bzw. Nenndurchmesser der Knochenschraube (1) konstant ist.

10. Osteosynthesevorrichtung nach Anspruch 8 oder 9, **dadurch gekennzeichnet, daß** sich der Schraubenkern (4) bis zum Außen- bzw. Nenndurchmesser der Knochenschraube (1) erweitert.

11. Osteosynthesevorrichtung nach einem der vorhergehenden Ansprüche, **dadurch gekennzeichnet, daß** die Breite der Gewindeschneide (7) der Knochenschraube (1) von der Schraubenspitze (5) ausgehend zunimmt.

12. Osteosynthesevorrichtung nach einem der vorhergehenden Ansprüche, **dadurch gekennzeichnet, daß** die Gewindesteigung konstant ist.

13. Osteosynthesevorrichtung nach einem der vorhergehenden Ansprüche, **dadurch gekennzeichnet, daß** das an den Schraubenkern (4) sich anschließende Ende des Gabelkopfes (3) ballig oder kugelig ausgebildet ist.

## Claims

1. An osteosynthesis device having a bone screw (1) or a pedicle screw, a fork head (3) that has a groove (12) and a corrective pin (26) seated in the groove (12) of the fork head (3), and an outside thread (17) for a head nut (18) at the fork head (3), **characterized in that** the bottom (13) of the groove (12) of the fork head (3) is provided with a plurality of longitudinal slots (14) lying parallel to one another and extending in longitudinal direction of the grooves (12), wherein the slots (14) are embodied without undercuts in the direction of the axis of the bone screw (1).

2. The osteosynthesis device as defined by claim 1, **characterized in that** the slots (14) are provided at the edge (15) of the groove of the fork head (3).

3. The osteosynthesis device as defined by one of the foregoing claims, **characterized in that** the corrective pin (26) is provided with longitudinal grooves (27), wherein the longitudinal grooves (27) correspond to the longitudinal slots (14) and extend in the axial direction.

4. The osteosynthesis device as defined by one of the foregoing claims, **characterized in that** the head nut (18) has an inside thread (20) with a centering collar (19).

5. The osteosynthesis device as defined by one of the foregoing claims, **characterized in that** the head nut (18) is embodied as a cap nut and has a coaxial inside thread (21) for a fastening screw (23).

6. The osteosynthesis device as defined by claim 5, **characterized in that** the fastening screw (23) has a ringlike knife edge (25) at the screwed-in end and is embodied in particular as a hexagonal socket screw.

7. The osteosynthesis device as defined by one of the foregoing claims, **characterized in that** the axial face end of the head nut (18) is embodied to be ball-shaped or particularly spherical, and e.g. is encompassed in particular by a hexagon (22).

8. The osteosynthesis device as defined by one of the foregoing claims, **characterized in that** the bone screw (1) has a screw body (4) that extends conically from the screw point (5), in the direction of the fork head (3).

9. The osteosynthesis device as defined by one of the foregoing claims, **characterized in that** the outside or nominal diameter of the bone screw (1) is constant.

10. The osteosynthesis device as defined by claim 8 or 9, **characterized in that** the screw body (4) widens as far as the outer or nominal diameter of the bone screw (1).

11. The osteosynthesis device as defined by one of the foregoing claims, **characterized in that** the width of the thread (7) of the bone screw (1) increases starting from the screw point (5).

12. The osteosynthesis device as defined by one of the foregoing claims, **characterized in that** the thread pitch is constant.

13. The osteosynthesis device as defined by one of the foregoing claims, **characterized in that** the end of the fork head (3) following the screw body (4) is embodied to be ball-shaped or particularly spherical.

## Revendications

1. Dispositif de synthèse de l'os comportant une vis d'os (1) ou une vis pédiculaire, avec une tête à fourche (3) présentant une rainure (12), une tige d'ajustement (26) logée dans la rainure (12) de la tête à fourche (3) et un pas de vis extérieur (17) pour un écrou à tête (18) sur la tête à fourche (3), **caractérisé en ce que** le fond (3) de la rainure (12) de la tête à fourche (3) est pourvu de plusieurs stries (14) disposées parallèlement les unes aux autres et s'étendant dans la direction longitudinale de la rainure (12), les stries ne présentant pas de partie formant rebord vers l'arrière dans la direction de l'axe de la vis d'os (1).

2. Dispositif d'ostéosynthèse selon l'une des revendications précédentes, **caractérisé en ce que** les stries (14) sont disposées au bord (15) du fond de la rainure de la tête à fourche (3).

3. Dispositif d'ostéosynthèse selon l'une des revendications précédentes, **caractérisé en ce que** la tige d'ajustement (26) comporte des rainures (27) dans le sens de la longueur, qui correspondent aux stries longitudinales (14) et disposées dans la direction de l'axe.

4. Dispositif d'ostéosynthèse selon l'une des revendications précédentes, **caractérisé en ce que** l'écrou à tête (18) comporte un pas de vis intérieur (20) avec une collerette de centrage (19).

5. Dispositif d'ostéosynthèse selon l'une des revendications précédentes, **caractérisé en ce que** l'écrou à tête (18) en forme d'écrou-chapeau comporte un pas de vis intérieur (21) coaxial pour une vis de fixation (23).

6. Dispositif d'ostéosynthèse selon la revendication 6, **caractérisé en ce que** la vis sans fixation (23) comporte une tranchée arrondie (25) à l'extrémité vissée, et en ce qu'elle est en forme par exemple de vis hexagonale intérieure à six pans creux.

7. Dispositif d'ostéosynthèse selon l'une des revendications précédentes, **caractérisé en ce que** la face frontale axiale de l'écrou à tête (18) est de forme convexe ou sphérique et présente par exemple une forme extérieure hexagonale (22).

8. Dispositif d'ostéosynthèse selon l'une des revendications précédentes, **caractérisé en ce que** la vis d'os (1) comporte une tige filetée conique (4) qui s'élargit depuis la pointe de la vis (5) en direction de la tête à fourche (3).

9. Dispositif d'ostéosynthèse selon l'une des revendications précédentes, **caractérisé en ce que** le diamètre extérieur ou nominal de la vis d'os (1) est constant.

10. Dispositif d'ostéosynthèse selon la revendication 9 ou la revendication 10, **caractérisé en ce que** la tige filetée (4) s'élargit jusqu'au diamètre nominal ou extérieur de la vis d'os (1).

11. Dispositif d'ostéosynthèse selon l'une des revendications précédentes, **caractérisé en ce que** la largeur du pas de vis (7) de la vis d'os (1) augmente depuis la pointe de la vis (5).

12. Dispositif d'ostéosynthèse selon l'une des revendications précédentes, **caractérisé en ce que** le pas de vis est constant.

13. Dispositif d'ostéosynthèse selon l'une des revendications précédentes, **caractérisé en ce que** l'extrémité de la tête à fourche (3) reliée à la tige de vis (4) est convexe ou sphérique.
